# EUROPEAN PATENT APPLICATION

(11) **EP 4 289 945 A1**
(43) Date of publication of application: **13.12.2023**
(21) Application number: 21928189.6
(22) Date of filing: 15.06.2021
(51) Int. Cl.: C12N 7/00, A23K 10/16, A23K 20/195, A01N 63/40, A61K 35/76, A61P 31/04, A61P 31/02, C02F 1/68, C11D 3/38

(54) **NOVEL BACTERIOPHAGE HAVING CLOSTRIDIUM PERFRINGENS-SPECIFIC BACTERICIDAL EFFECT AND ANTIBACTERIAL COMPOSITION COMPRISING SAME**

(30) Priority: 25.02.2021 KR 20210025978
(71) Applicant: CJ Cheiljedang Corporation, Seoul 04560 (KR)
(72) Inventor: JEON, Jong Soo, Seoul 04560 (KR); KIM, Ji Eun, Seoul 04560 (KR); MOON, Jun Ok, Seoul 04560 (KR); CHAE, Jong Pyo, Seoul 04560 (KR); KIM, Yu Jin, Seoul 04560 (KR); LEE, Seung Eun, Seoul 04560 (KR)
(74) Representative: Icosa
(86) International application number: PCT/KR2021/007484
(87) International publication number: WO 2022/181894

(57) **Abstract**

The present disclosure relates to a novel bacteriophage having ability to specifically kill *Clostridium perfringens* and an antibacterial composition comprising the same. The novel bacteriophage CJ_CP_20-25 has the effect of specifically killing *Clostridium perfringens,* exhibits excellent acid resistance and heat resistance, and can thus be widely used in antibiotics, feed additives, drinking water additives, feed, drinking water, disinfectants, or cleaning agents for the prevention or treatment of infectious diseases caused by *Clostridium perfringens.*

## Description

### [Technical Field]

The present disclosure relates to a novel bacteriophage having ability to specifically kill *Clostridium perfringens* and an antibacterial composition comprising the same.

### [Background Art]

*Clostridium perfringens* is known as a Gram-positive, large obligate anaerobic bacillus that does not have flagella and forms spores. *Clostridium perfringens* is a bacterium that causes diarrhea and the like in animals, especially in livestock including chickens and pigs, and is recognized as one of the most important and deadly pathogens in the livestock industry as well as poultry typhus caused by *Salmonella.*

Currently, necrotic enteritis caused by *Clostridium perfringens* infection, which is one of the most frequently occurring diseases in the poultry and pig industries, is a disease that causes severe necrotic lesions in the lower small intestine of chickens or pigs and diarrhea with blood as the main symptoms. Such necrotic enteritis causes dehydration and intermittent diarrhea symptoms in infected animals depending on the severity of disease, gradually weakens the animal's body, and causes growth disorders, and is thus a disease that has become a significant problem in the livestock industry. Moreover, *Clostridium perfringens,* a cause of necrotic enteritis, easily spreads through animal feces, is thus easily transmitted between animals in a common breeding space by oral infection and the like through soil or contaminated feed, and is a severe problem especially in young livestock because of its high incidence.

In the field of livestock industry and the like, in order to prevent and treat diseases caused by bacterial infection of livestock, various antibiotics have been used, but there are currently a number of restrictions on the administration of antibiotics around the world as problems have emerged such as misuse of or resistance to antibiotics and the possibility that antibiotics remain in animals.

Accordingly, bacteriophages, which are bacteria-specific viruses that inhibit bacterial growth by infecting specific bacteria, have recently been studied as new alternatives to antibiotics since bacteriophages exhibit stronger host specificity than antibiotics, but their number is still absolutely insufficient. Therefore, in order to prevent and treat infectious diseases caused by *Clostridium perfringens,* which are an important problem in the livestock industry including pigs and poultry, it is continuously required that new bacteriophages be developed which have a broad bacteriolytic spectrum and excellent acid resistance and heat resistance and thus have high industrial applicability.

### [Citation List]

### [Patent Literature]

[Patent Literature 1] US 6,942,858 B1

### [Disclosure]

### [Technical Problem]

An object of the present disclosure is to provide a bacteriophage CJ_CP_20-25 that has ability to specifically kill *Clostridium perfringens* and is deposited with accession number KCCM12934P, a composition, antibiotic, feed additive, drinking water additive, disinfectant, or cleaning agent for prevention or treatment of infectious diseases caused by *Clostridium perfringens* comprising the same as an active ingredient, feed comprising the feed additive, or drinking water comprising the drinking water additive.

Another object of the present disclosure is to provide a method for preventing or treating infectious diseases caused by *Clostridium perfringens* using the bacteriophage CJ_CP_20-25 or a composition for prevention or treatment of infectious diseases caused by *Clostridium perfringens* comprising the same as an active ingredient.

Still another object of the present disclosure is to provide use of the bacteriophage CJ_CP_20-25 or a composition for prevention or treatment of infectious diseases caused by *Clostridium perfringens* comprising the same as an active ingredient for the preparation of prophylactic or therapeutic agents for infectious diseases caused by *Clostridium perfringens.*

### [Technical Solution]

Each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description below. Further, those skilled in the art will recognize, or be able to ascertain using no more than routine experimentation, many equivalents to the specific aspects of the present disclosure described herein. Further, these equivalents should be interpreted to fall within the scope of the present disclosure.

An aspect provides a bacteriophage CJ_CP_20-25 that has ability to specifically kill *Clostridium perfringens* and is deposited with accession number KCCM12934P.

As used herein, the term *"Clostridium perfringens* (CP)" refers to a Gram-positive anaerobic bacillus, which corresponds to a causative organism causing infectious diseases in animals such as chickens and pigs.

As used herein, the term "bacteriophage" refers to a bacterium-specific virus that infects a specific bacterium and inhibits the growth of the bacterium, and refers to a virus containing single or double-stranded DNA or RNA as a genetic material.

Another aspect provides a composition for prevention or treatment of infectious diseases caused by *Clostridium perfringens* comprising the bacteriophage CJ_CP_20-25 as an active ingredient.

The bacteriophage CJ_CP_20-25 is as described above.

The bacteriophage CJ_CP_20-25 has ability to specifically kill *Clostridium perfringens,* and thus suppresses infection with *Clostridium perfringens* by killing *Clostridium perfringens* introduced into an individual when administered to the individual prior to the onset of infectious diseases caused by *Clostridium perfringens,* whereby it is possible to prevent infectious diseases caused by *Clostridium perfringens.* In addition, when the bacteriophage CJ_CP_20-25 is administered to an individual having an infectious disease caused by *Clostridium perfringens,* the bacteriophage CJ_CP_20-25 can alleviate or eliminate the symptoms caused by *Clostridium perfringens* by killing *Clostridium perfringens* present in the individual, whereby it is possible to treat infectious diseases caused by *Clostridium perfringens.*

As used herein, the term "prevention" may refer to any action that inhibits or delays the onset of a disease in an individual by administration of the bacteriophage or composition, and the term "treatment" may refer to any action that allows the symptoms of a disease in an individual to improve, be alleviated, or disappear due to administration of the bacteriophage or composition.

The infectious diseases caused by *Clostridium perfringens* may be, for example, necrotic enteritis, but is not limited thereto.

As used herein, the term "necrotic enteritis" refers to one of the infectious diseases caused by *Clostridium perfringens,* and corresponds to a bacterial disease that frequently occurs in livestock such as broilers and causes considerable damage. Symptoms of necrotic enteritis are caused by excessive proliferation of *Clostridium perfringens* in the small intestine, and include necrosis of the digestive mucosa and sudden diarrhea. For example, super-acute necrotic enteritis in pigs causes death 1 to 2 days after the onset, and acute necrotic enteritis causes diarrhea with blood and then death after 2 to 3 days. In addition, sub-acute necrotic enteritis causes continuous diarrhea (without bloody stool) for 5 to 7 days and then weakness and dehydration, and chronic necrotic enteritis may cause intermittent diarrhea and growth disorders.

The composition for prevention or treatment of infectious diseases caused by *Clostridium perfringens* may comprise the bacteriophage CJ_CP_20-25 at 1 × 10² PFU/mL to 1 × 10¹² PFU/mL or 1 × 10⁵ PFU/g to 1 × 10¹⁰ PFU/g.

The composition for prevention or treatment of infectious diseases caused by *Clostridium perfringens* may further comprise a pharmaceutically acceptable carrier, and may be formulated with the carrier and provided as a food, drug, feed additive, drinking water additive, or the like.

As used herein, the term "pharmaceutically acceptable carrier" may refer to a carrier or diluent that does not stimulate the organism and does not inhibit the biological activity and properties of the administered compound.

The kind of carrier that may be contained in the composition is not particularly limited, and any carrier that is commonly used in the art and pharmaceutically acceptable may be used. Non-limiting examples of the carrier include saline, sterile water, Ringer's solution, buffered saline, albumin injection solution, dextrose solution, maltodextrin solution, and glycerol. These may be used singly or in mixture of two or more kinds thereof, and other conventional additives such as antioxidants, buffers, and bacteriostats may be added if necessary. In addition, diluents, dispersants, binders, and lubricants may be optionally added to form injectable formulations such as aqueous solutions, suspensions, or emulsions or pills, capsules, granules, or tablets.

The composition for prevention or treatment of infectious diseases caused by *Clostridium perfringens* may be orally or parenterally administered, and a method to apply or spray the composition to a diseased site may also be used. For parenteral administration, intravenous administration, intraperitoneal administration, intramuscular administration, subcutaneous administration, or local administration may be used. Suitable application, spraying, and dosage of the composition vary depending on factors such as formulation method, mode of administration, the age, weight, sex, severity of disease symptoms, and food of the target animal and patient, administration time, administration route, excretion rate and response sensitivity, and an ordinarily skilled physician or veterinarian can readily determine and prescribe a dosage effective for the desired treatment.

Formulations for oral administration of the composition for prevention or treatment of infectious diseases caused by *Clostridium perfringens* may include, for example, tablets, troches, lozenges, aqueous or oily suspensions, powders or granules, emulsions, hard or soft capsules, and syrup or elixirs. In order to prepare the composition into formulations such as tablets and capsules, binders such as lactose, saccharose, sorbitol, mannitol, starch, amylopectin, cellulose, or gelatin; excipients such as dicalcium phosphate; disintegrants such as corn starch or sweet potato starch; and lubricating oils such as magnesium stearate, calcium stearate, sodium stearyl fumarate, or polyethylene glycol wax may be contained. In the case of capsule formulation, liquid carriers such as fatty oil may be further contained in addition to the above-mentioned substances.

Formulations for parenteral administration of the composition for prevention or treatment of infectious diseases caused by *Clostridium perfringens* may include injection forms such as subcutaneous injection, intravenous injection or intramuscular injection; suppository injection forms; or spraying forms such as aerosol that can be inhaled through the respiratory tract. In order to prepare the composition into an injectable formulation, the composition of the present invention may be mixed with a stabilizer or buffering agent in water to prepare a solution or suspension, and this may be formulated for unit administration in ampoules or vials. In the case of formulation for spraying such as aerosol, a propellant, or the like may be blended with additives so that the water-dispersed concentrate or wet powder is dispersed.

The composition for prevention or treatment of infectious diseases caused by *Clostridium perfringens* may comprise a preservative, a stabilizer, a wetting or emulsifying agent, a cryoprotectant, an excipient, or the like.

The preservative, stabilizer, or excipient may be contained in the composition in an effective amount sufficient to reduce deterioration of the composition.

As used herein, the term "deterioration" may include a decrease in the number of bacteria of the bacteriophage CJ_CP_20-25 contained in the composition, a decrease in activity of the bacteriophage CJ_CP_20-25, or a combination thereof.

For example, as the composition comprises a preservative, stabilizer, or excipient in an effective amount sufficient to reduce deterioration of the composition, the bacteriophage CJ_CP_20-25 in the composition may survive or maintain its activity for a longer period of time unlike bacteriophages existing in nature.

As the preservative, stabilizer, or excipient, those commonly used in the art may be used without limitation as long as they can reduce deterioration of the composition. For example, the stabilizer may be any one or more selected from the group consisting of alginates, sodium alginate, casein, sodium casein, cellulose, methylcellulose, carboxymethylcellulose, sodium carboxymethylcellulose, pectin, gelatin, gum arabic, xanthan gum, dextran, cyclodextrin, and starch.

The cryoprotectant may be contained in the composition in an effective amount sufficient to reduce deterioration of the composition when the composition is in a lyophilized state. For example, unlike bacteriophages existing in nature so that the bacteriophages exhibit decreased activity or cannot survive in a lyophilized composition, the bacteriophage may maintain its activity or survive even in the lyophilized composition as the composition comprises a cryoprotectant in an effective amount sufficient to reduce deterioration of the composition in a lyophilized state.

As the cryoprotectant, those commonly used in the art may be used without limitation as long as they are to reduce deterioration of the composition in a lyophilized state. For example, the cryoprotectant may be any one or more selected from the group consisting of glycerol, ethylene glycol, propylene glycol, dimethyl sulfoxide (DMSO), glucose, trehalose, maltodextrin, dextrin, skim milk, and starch.

Another aspect provides an antibiotic comprising the bacteriophage CJ_CP_20-25 as an active ingredient.

The bacteriophage CJ_CP_20-25 is as described above.

As used herein, the term "antibiotic" refers to an agent capable of killing bacteria or inhibiting the growth of bacteria, and may be a generic term for preservatives, bactericides, and antibacterial agents.

The antibiotic comprising the bacteriophage CJ_CP_20-25 as an active ingredient exhibits significantly high specificity for *Clostridium perfringens* compared to conventional antibiotics and can thus kill only specific pathogens without killing beneficial bacteria, and the antibiotic does not induce drug resistance and may thus exhibit an effect of having extended product lifespan compared to conventional antibiotics.

The antibiotic may be prepared by a manufacturing method commonly used in the art.

The antibiotic may comprise an additive in an effective amount sufficient to reduce degradation of the antibiotic, and the additive may be a preservative, a stabilizer, an excipient, a cryoprotectant, or the like. As the antibiotic comprises the additive, the bacteriophage CJ_CP_20-25 in the antibiotic may survive or maintain its activity for a longer period of time unlike bacteriophages existing in nature.

As the preservative, stabilizer, excipient, or cryoprotectant, those commonly used in the art may be used without limitation as long as they can reduce deterioration of the antibiotic.

Another aspect provides a feed additive comprising the bacteriophage CJ_CP_20-25 as an active ingredient. Still another aspect provides feed comprising the feed additive. Still another aspect provides a drinking water additive comprising the bacteriophage CJ_CP_20-25 as an active ingredient. Still another aspect provides drinking water comprising the drinking water additive.

The bacteriophage CJ_CP_20-25 is as described above.

The feed additive or drinking water additive may be prepared in the form of a composition comprising the bacteriophage CJ_CP_20-25 and mixed into feed or drinking water, or may be used in such a way that the bacteriophage CJ_CP_20-25 is directly added when feed or drinking water is manufactured.

The bacteriophage CJ_CP_20-25 to be contained in the feed additive or drinking water additive may be in a liquid state or a dried solid state, may specifically be in the form of a dry powder. The drying method of the bacteriophage CJ_CP_20-25 may be ventilation drying, natural drying, spray drying, or freeze drying, but is not limited thereto. The bacteriophage CJ_CP_20-25 may be mixed in the form of a dry powder at 0.05% to 10% by weight, specifically 0.1% to 2% by weight based on the weight of the feed additive or drinking water additive.

The feed additive or drinking water additive comprising the bacteriophage CJ_CP_20-25 as an active ingredient may further comprise other additives if necessary. Non-limiting examples of the additives that can be used include, but are not limited to, binders, emulsifiers, preservatives, and the like that are added to prevent deterioration of feed or drinking water; and amino acids, vitamins, enzymes, probiotics, flavoring agents, non-protein nitrogen compounds (NPN), silicate agents, buffering agents, colorants, extractants, or oligosaccharides that are added to increase the utility of feed or drinking water, and these may be added singly, or two or more kinds thereof may be added together.

As raw materials contained in the feed or drinking water in addition to the bacteriophage CJ_CP_20-25, those commonly used in the art may be used without limitation. Non-limiting examples of the feed include plant feed such as grains, root fruits, food processing by-products, algae, fibers, pharmaceutical by-products, oils and fats, starches, gourd or grain by-products; and animal feed such as proteins, inorganic materials, oils and fats, minerals, single-cell proteins, zooplankton or food. These may be used singly or in mixture of two or more kinds thereof.

The feed additive may be contained at 0.05 to 10 parts by weight, for example, 0.1 to 2 parts by weight based on 100 parts by weight of feed. The drinking water additive may be contained at 0.0001 to 0.01 parts by weight, for example, 0.001 to 0.005 parts by weight based on 100 parts by weight of drinking water.

The feed additive, feed, drinking water additive or drinking water may be prepared by a manufacturing method commonly used in the art.

The feed additive, feed, drinking water additive or drinking water may comprise an additive in an effective amount sufficient to reduce deterioration thereof, and the additive may be a preservative, a stabilizer, an excipient, or a cryoprotectant. As the feed additive, feed, drinking water additive or drinking water comprises the additive, the bacteriophage CJ_CP_20-25 in the feed additive, feed, drinking water additive or drinking water may survive or maintain its activity for a longer period of time unlike bacteriophages existing in nature. As the preservative, stabilizer, excipient, or cryoprotectant, those commonly used in the art may be used without limitation as long as they can reduce deterioration of the feed additive, feed, drinking water additive or drinking water.

Another aspect provides a disinfectant comprising the bacteriophage CJ_CP_20-25 as an active ingredient. Still another aspect provides a cleaning agent comprising the bacteriophage CJ_CP_20-25 as an active ingredient.

The bacteriophage CJ_CP_20-25 is as described above.

The formulation of the disinfectant or cleaning agent is not particularly limited, and the disinfectant or cleaning agent may be used by being prepared into a formulation commonly known in the art. The disinfectant or cleaning agent may be prepared by a manufacturing method commonly used in the art.

The disinfectant may be sprayed to remove *Clostridium perfringens,* and may be sprayed on an animal's activity area, slaughterhouse, death area, cooking place, cooking equipment, or the like, but is not limited thereto.

The cleaning agent may be used for cleaning the skin surface or body parts of animals exposed or likely to be exposed to *Clostridium perfringens,* but is not limited thereto.

The disinfectant or cleaning agent may comprise an additive in an effective amount sufficient to reduce deterioration thereof, and the additive may be a preservative, a stabilizer, an excipient, or a cryoprotectant. As the disinfectant or cleaning agent comprises the additive, the bacteriophage CJ_CP_20-25 in the disinfectant or cleaning agent may survive or maintain its activity for a longer period of time unlike bacteriophages existing in nature. As the preservative, stabilizer, excipient, or cryoprotectant, those commonly used in the art may be used without limitation as long as they can reduce deterioration of the disinfectant or cleaning agent.

Another aspect provides a method for preventing or treating infectious diseases caused by *Clostridium perfringens,* which comprises administering the bacteriophage CJ_CP_20-25 or a composition for prevention or treatment of infectious diseases caused by *Clostridium perfringens* comprising the same as an active ingredient to an animal other than a human.

The bacteriophage CJ_CP_20-25, the composition for prevention or treatment of infectious diseases caused by *Clostridium perfringens* comprising the same as an active ingredient, and the infectious diseases caused by *Clostridium perfringens* are as described above. Specifically, the infectious diseases caused by *Clostridium perfringens* may be, for example, necrotic enteritis.

The prevention or treatment method specifically comprises administering the bacteriophage CJ_CP_20-25 or a composition comprising the same as an active ingredient to an animal other than a human infected with or at risk of being infected with *Clostridium perfringens* in a pharmaceutically effective amount. The bacteriophage CJ_CP_20-25 or a composition comprising the same as an active ingredient may be administered to an animal in the form of a pharmaceutical preparation, or may be administered by being mixed with animal feed or drinking water in the form of a feed additive or drinking water additive and fed.

The bacteriophage CJ_CP_20-25 or a composition comprising the same as an active ingredient may be administered through various oral or parenteral routes as long as it can reach the target tissue, may specifically be administered in a conventional manner through oral, rectal, topical, intravenous, intraperitoneal, intramuscular, intraarterial, transdermal, intranasal, and inhalation routes and the like.

A suitable total daily usage of the bacteriophage CJ_CP_20-25 or a composition comprising the same as an active ingredient may be determined by a treating physician within the scope of sound medical judgment, and this is apparent to those of ordinary skill in the art.

The specific pharmaceutically effective amount of the bacteriophage CJ_CP_20-25 or a composition comprising the same as an active ingredient for a particular animal may be determined in consideration of the kind and extent of the response to be achieved and the age, weight, general health status, sex or diet of the individual as well as the administration time and administration route of the bacteriophage CJ_CP_20-25 or a composition comprising the same as an active ingredient and the secretion rate of the composition, the treatment period, and the like, and may vary depending on a number of factors, including drugs or other components of the composition used simultaneously or at the same time, and similar factors well known in the pharmaceutical field.

The animal other than a human is not particularly limited as long as it is an animal that may be infected with *Clostridium perfringens.* For example, the animal may be birds and mammals, and may specifically include chickens, ducks, pigs, and the like, but is not limited thereto.

Another aspect provides use of the bacteriophage CJ_CP_20-25 or a composition for prevention or treatment of infectious diseases caused by *Clostridium perfringens* comprising the same as an active ingredient for the preparation of prophylactic or therapeutic agents for infectious diseases caused by *Clostridium perfringens.*

The bacteriophage CJ_CP_20-25, the composition for prevention or treatment of infectious diseases caused by *Clostridium perfringens* comprising the same as an active ingredient, and the infectious diseases caused by *Clostridium perfringens* are as described above. Specifically, the infectious diseases caused by *Clostridium perfringens* may be, for example, necrotic enteritis.

### [Advantageous Effects]

A novel bacteriophage CJ_CP_20-25 has the effect of specifically killing *Clostridium perfringens,* exhibits excellent acid resistance and heat resistance, and can thus be widely used in antibiotics, feed additives, drinking water additives, feed, drinking water, disinfectants or cleaning agents for the prevention or treatment of infectious diseases caused by *Clostridium perfringens.*

### [Brief Description of Drawings]

FIG. 1 is an electron micrograph of a novel bacteriophage CJ_CP_20-25;
FIG. 2 is a graph illustrating the result of confirming the pH stability of a novel bacteriophage CJ_CP_20-25; and
FIG. 3 is a graph illustrating the result of confirming the stability of a novel bacteriophage CJ_CP_20-25 at 60°C.

### [Detailed Description of the Invention]

Hereinafter, the present invention will be described in more detail with reference to Examples. However, these Examples are for illustrative purposes of one or more embodiments, and the scope of the present invention is not limited to these Examples.

### Example 1. Isolation of bacteriophage having ability to kill Clostridium perfringens (CP)

### Example 1-1. Preparation of pretreated fecal sample solution

Fecal samples of pigs, poultry, and cattle were collected from livestock farms in Seoul, Gyeonggi-do, Chungcheong-do, and Gyeongsang-do, Korea and 20 g of each sample was diluted with 80 mL of PBS and centrifuged at 10,000 rpm for 15 minutes. After the supernatant was filtered through a 0.2 µm filter, 10% (w/v) aqueous sodium chloride solution was added to the filtrate, and the mixture was stored at 4°C for 12 hours. Thereafter, 10% (w/v) polyethylene glycol 8000 (Sigma-Aldrich, Cat. No. P2139) was added thereto, and the mixture was stored at 4°C for 12 hours, and then the supernatant was removed by centrifugation at 15,000 rpm for 1 hour for concentration. The precipitate was dissolved in 10 mL of SM buffer (5.8 g/L sodium chloride, 2 g/L MgSO₄·7H₂O, 0.05 M Tris-CI (pH 7.5)), the solution was filtered through a 0.2 µm filter, and the filtrate was stored at 4°C.

### Example 1-2. Preparation of bacteriophage concentrate

CP strains isolated from fecal samples collected from livestock farms in Seoul, Gyeonggi-do, Chungcheong-do, and Gyeongsang-do, Korea and CP strains distributed from the Animal and Plant Quarantine Agency were inoculated into 2 mL of BHI (brain heart infusion) medium, and standing-cultured at 42°C for 18 hours under an anaerobic condition. Thereafter, 1 mL of the CP strain culture solution and 1 mL of the pretreated fecal sample solution obtained in Example 1-1 were inoculated into 50 mL of BHI medium and mixed standing-cultured at 42°C for 18 hours. After centrifugation of the mixed culture solution at 6,000 rpm for 20 minutes, the supernatant was filtered through a 0.2 µm filter, 10% (w/v) polyethylene glycol 8000 was added thereto, and the mixture was stored at 4°C for 12 hours. Thereafter, the supernatant was removed by centrifugation at 15,000 rpm for 1 hour for concentration, the precipitate was dissolved in 1 mL of SM buffer, the solution was filtered through a 0.2 µm filter, and the filtrate was stored at 4°C.

### Examples 1-3. Screening and isolation of bacteriophage

Mixed were 50 µL of the bacteriophage concentrate prepared in Example 1-2, 5 mL of 0.7% (w/v) agar (BD DIFCO, Cat. No. 44164), and 50 µL of a culture solution prepared by shaking-culturing the same CP strain as that used in Example 1-2 so as to have an absorbance (O.D.) of 2 at 600 nm, and a double-layer agar plaque assay was performed using a BHI plate medium with a diameter of 150 mm. The plaque formed on soft agar was punched with a 200 µL tip, put in 0.5 mL of SM buffer, and eluted. The solution containing the eluted bacteriophage was repeatedly subjected to the double-layer agar plaque assay until single plaques of the same type were formed to isolate a solution containing a pure bacteriophage. The obtained bacteriophage-containing solution was filtered through a 0.2 µm filter, 10% (w/v) polyethylene glycol 8000 was added thereto, and the mixture was stored at 4°C for 12 hours. Thereafter, the supernatant was removed by centrifugation at 15,000 rpm for 1 hour for concentration, and the precipitate was dissolved in 1 mL of SM buffer, the solution was filtered through a 0.2 µm filter, and the filtrate was stored at 4°C.

### Example 2. Whole genome sequencing (WGS) analysis of isolated bacteriophage

DNA was extracted from 1 mL of the bacteriophage concentrate purely isolated in Example 1-3 using the CsCI gradient method and phage DNA isolation kit (Norgen Biotek-Corp. Kit, Cat. No. 46800). The whole genome sequencing analysis was performed by Macrogen Inc., the genes were combined using De novo assembly software (SPAdes 3.13.0), and the open reading frame (ORF) was performed using GeneMark.hmm and NCBI ORF finder. The function of each ORF was annotated using BLASTP (E values of <0.1) and PSI-BLAST (E value of <0.005) programs.

As a result, the isolated bacteriophage has been confirmed to have a nucleotide sequence of SEQ ID NO: 1 with 51,670 bp, 73 ORF, and 34.10% G + C content, and this has 97% sequence identity with the previously reported *Clostridium* phage CP3 (MF001357.1), but it has been confirmed that there is no bacteriophage of which all fragments are 100% identical with those of the previously reported *Clostridium* phage CP3 (MF001357.1), and it has been thus found that the bacteriophage is a newly isolated bacteriophage. Accordingly, the novel bacteriophage was named bacteriophage CJ_CP_20-25, and was deposited at the Korean Culture Center of Microorganisms, an international depository under the Budapest Treaty as of January 18, 2021, and was given an accession number KCCM12934P.

### Example 3. Morphological analysis of bacteriophage CJ_CP_20-25

To obtain a high-purity bacteriophage solution, a CsCI gradient method was performed. Specifically, CsCI solutions, which were dissolved in SM buffer and each have a density of 1.7, 1.5, 1.45, or 1.3 were prepared, and the CsCI solutions were dispensed in a 15 mL ultracentrifuge tube (Beckman Coulter, Cat. No. Z00901SCA) by 2 mL so as to form layers from higher-density layers to lower-density layers, and 2 mL of the bacteriophage CJ_CP_20-25 concentrate obtained in Example 1-3 was dispensed at the top. This was centrifuged at 4°C and 25,000 rpm for 2 hours, and then only the white bacteriophage layer formed in the tube was collected with a syringe (Satorius, Cat. No. 17822-K). The collected bacteriophage solution was dropped on a carbon-coated copper grid by 1 µL and then stained with 2% uranyl acetate for 15 seconds, and the shape was observed under an electron microscope (TEM, JEOL JEM-101, Tokyo, Japan).

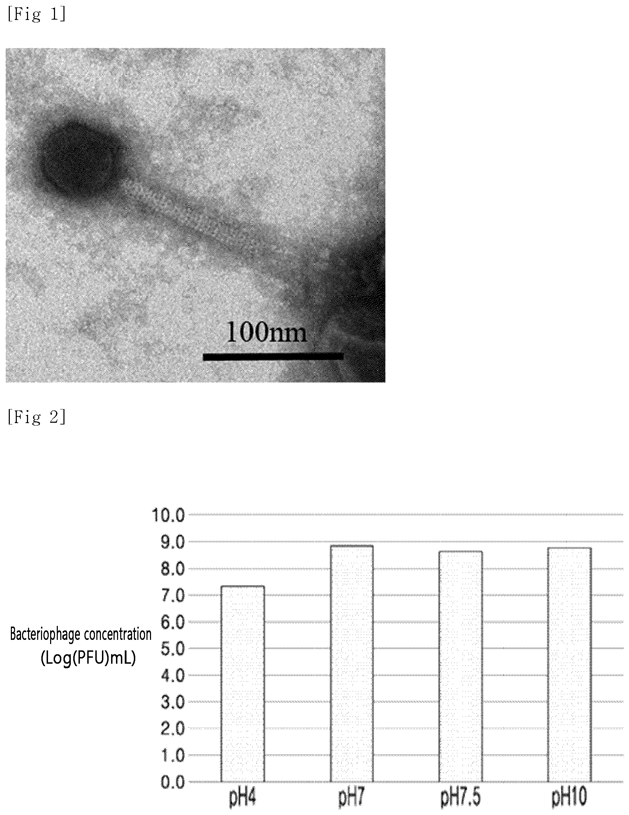

As a result, as illustrated in FIG. 1, the bacteriophage CJ_CP_20-25 has been observed to have morphologically an icosahedral head and a contractile tail with a length of about 100 nm, and thus found to belong to the family Myoviridae, order Caudovirales.

### Example 4. Evaluation on pH stability of bacteriophage CJ_CP_20-25

In order to examine whether the bacteriophage CJ_CP_20-25 is stable over a wide pH range, solutions each having a pH of 4, 7, 7.5, or 10 (pH 4: 0.2 M sodium acetate solution; pH 7 and 7.5: 0.2 M sodium phosphate solutions; and pH 10: 0.2 M Tris-HCl solution) were prepared. After 450 µL of each solution having the corresponding pH and 50 µL of 2 × 10¹⁰ PFU/mL bacteriophage solution were mixed together and left to stand at 4°C for 2 hours, a double-layer agar plaque assay was performed to evaluate the increase or decrease in titer.

As a result, as illustrated in FIG. 2, the bacteriophage CJ_CP_20-25 has been found to be a bacteriophage exhibiting excellent acid resistance since the bacteriophage CJ_CP_20-25 is stable without losing activity at pH 7 to 10 and exhibits activity decreased by only about 1.3 logs compared to that of the pH 7.5 group even at pH 4.

### Example 5. Evaluation on thermal stability of bacteriophage CJ_CP_20-25

In order to examine whether the bacteriophage CJ_CP_20-25 is stable at a high temperature, 500 µL of 2 × 10⁸ PFU/mL bacteriophage solution was left to stand at 60°C for 0, 3, 6, or 24 hours, and a double-layer agar plaque assay was performed to evaluate the increase or decrease in titer.

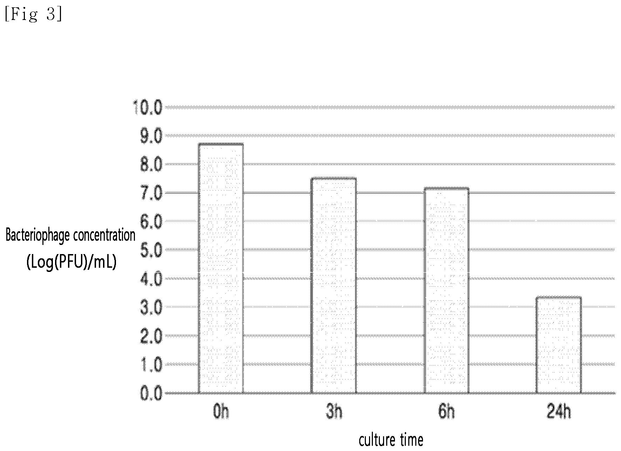

As a result, as illustrated in FIG. 3, the bacteriophage CJ_CP_20-25 has a decrease of about 1.2 logs in activity compared to the control group exposed for 0 hours when exposed at 60°C for 3 hours, a decrease of about 1.6 logs in activity compared to the control group when exposed for 6 hours, and a decrease of about 5.4 logs in activity compared to the control group but still exhibits activity even when exposed for 24 hours. Hence, the bacteriophage CJ_CP_20-25 has been found to be a bacteriophage exhibiting excellent heat resistance.

### Example 6. Evaluation on bacteriolytic spectrum of bacteriophage CJ_CP_20-25

In order to evaluate the bacteriolytic spectrum of the bacteriophage CJ_CP_20-25, a total of 45 types of CP strains, which were isolated from fecal samples collected from livestock farms in Seoul, Gyeonggi-do, Chungcheong-do, and Gyeongsang-do, Korea and distributed from the Animal and Plant Quarantine Agency, were cultured in BHI liquid medium, respectively. Thereafter, 50 µL of each of the strain culture solutions was inoculated into 5 mL of 0.7% soft agar, followed by pouring into a Petri dish, plating, and standing for 5 minutes. Thereafter, 10 µL of the bacteriophage CJ_CP_20-25 concentrate obtained in Example 1-3 was spotted on soft agar, followed by standing culture at 42°C for 18 hours. After the termination of culture, the bacteriolytic spectrum of bacteriophage CJ_CP_20-25 was evaluated according to the presence or absence of a plaque formed on the soft agar.

**[Table 1]**

| Name of CP strain | Formation of plaque | Name of CP strain | Formation of plaque |
|---|---|---|---|
| HLYS-1 | ++ | BCCP42-2 | ++ |
| HLYS-3 | ++ | BCCP43-1 | ++ |
| JSH-1 | ++ | BCCP47-2 | ++ |
| CP-KCCM 40947 | ++ | BCCP48-3 | ++ |
| KJW-2 | ++ | BCCP51-1-1 | ++ |
| CP-KJW-1 | ++ | BCCP52-2-8 | ++ |
| CP-JSH-1 | ++ | BCCP53-2-3 | ++ |
| CP-OYS-2 | ++ | BCCP54-3-8 | ++ |
| CP-BC-1 | ++ | BCCP55-3-1 | ++ |
| CP-BSW-4 | ++ | SBCCP429-2 | ++ |
| CP-HBM-2 | ++ | SBCCP321 | ++ |
| CP-HLYS | ++ | SBCCP343 | ++ |
| CP-KW-1 | ++ | SBCCP361 | ++ |
| CP-BS-1 | ++ | ELCCP6-1 | ++ |
| CP-HL-1 | ++ | OYS-2-1 | ++ |
| CP-LJN-1 | ++ | OYS-2-2 | ++ |
| BCCP17-1 | ++ | 1-1-2 | ++ |
| BCCP23-4 | ++ | 1-1 | ++ |
| BCCP37-2 | ++ | C3 | ++ |
| BCCP38-1 | ++ | CP-ATCC12921 | ++ |
| BCCP39-1 | ++ | CP-ATCC13124 | ++ |
| BCCP40-1 | ++ | CP-CCARM 18020 | ++ |
| BCCP41-3 | ++ | | |

| | | | |
|---|---|---|---|
| (++: Formation of clear plaque; +: Formation of turbid plaque) | | | |

As a result, as presented in Table 1, the bacteriophage CJ_CP_20-25 has been confirmed to have the ability to kill a wide range of CP strains since plaques have been formed in all 45 types of CP strains tested.

From the above description, those of ordinary skill in the art to which the present disclosure pertains will be able to understand that the present disclosure may be implemented in other specific forms without changing the technical spirit or essential characteristics thereof. In this regard, it should be understood that the embodiments described above are illustrative in all respects but not restrictive. The scope of the present disclosure is defined by the appended claims rather than by the description preceding them, and therefore all changes or modifications derived from the meaning and scope of the following claims and their equivalents should be construed as being included in the scope of the present disclosure.

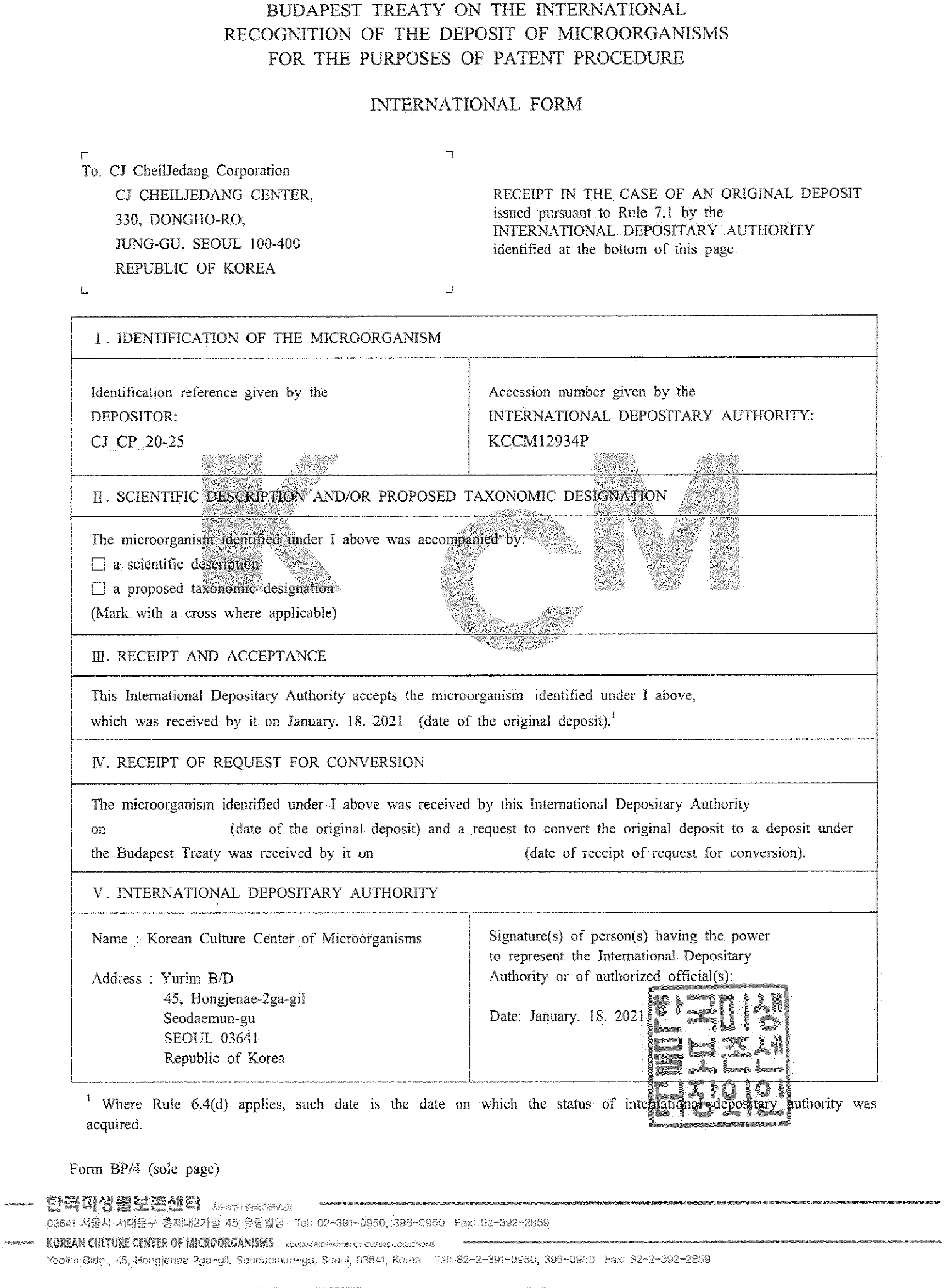
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

Ⅰ. IDENTIFICATION OF THE MICROORGANISM
Identification reference given by the
DEPOSITOR:
CJ CP 20-25 | Accession number given by the
INTERNATIONAL DEPOSITARY AUTHORITY:
KCCM12934P
Ⅱ. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
Ⅲ. RECEIPT AND ACCEPTANCE
This International Depositary Authority accepts the microorganism identified under I above,
which was received by it on January. 18. 2021 (date of the original deposit).¹
Ⅳ. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
Ⅴ. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 18. 2021

¹ Where Rule 6.4(d) applies, such date is the date on which the status of international depositary authority was
acquired.

Form BP/4 (sole page)

한국미생물보존센터 사단법인 한국종균협회
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A bacteriophage CJ_CP_20-25 that has ability to specifically kill *Clostridium perfringens* (CP) and is deposited with accession number KCCM12934P.

2. A composition for prevention or treatment of infectious diseases caused by *Clostridium perfringens* comprising the bacteriophage CJ_CP_20-25 according to claim 1 as an active ingredient.

3. The composition according to claim 2, wherein the infectious diseases caused by *Clostridium perfringens* are necrotic enteritis.

4. An antibiotic comprising the bacteriophage CJ_CP_20-25 according to claim 1 as an active ingredient.

5. A feed additive comprising the bacteriophage CJ_CP_20-25 according to claim 1 as an active ingredient.

6. Feed comprising the feed additive according to claim 5.

7. A drinking water additive comprising the bacteriophage CJ_CP_20-25 according to claim 1 as an active ingredient.

8. Drinking water comprising the drinking water additive according to claim 7.

9. A disinfectant comprising the bacteriophage CJ_CP_20-25 according to claim 1 as an active ingredient.

10. A cleaning agent comprising the bacteriophage CJ_CP_20-25 according to claim 1 as an active ingredient.

11. A method for preventing or treating infectious diseases caused by *Clostridium perfringens,* the method comprising administering the bacteriophage CJ_CP_20-25 according to claim 1 or the composition according to claim 2 to an animal other than a human.

12. The method according to claim 11, wherein the infectious diseases caused by *Clostridium perfringens* are necrotic enteritis.
